Europäisches Patentamt

European Patent Office

Office européen des brevets

(19)

(11) Numéro de publication: **0 500 460 B1**

(12) ## FASCICULE DE BREVET EUROPEEN

(45) Date de publication du fascicule du brevet: **06.09.95** (51) Int. Cl.⁶: **A47L 13/17**

(21) Numéro de dépôt: **92400438.5**

(22) Date de dépôt: **19.02.92**

(54) **Combiné de nettoyage et procédés de fabrication.**

(30) Priorité: **19.02.91 FR 9101963**

(43) Date de publication de la demande:
**26.08.92 Bulletin 92/35**

(45) Mention de la délivrance du brevet:
**06.09.95 Bulletin 95/36**

(84) Etats contractants désignés:
**DE ES FR GB IT**

(56) Documents cités:
**EP-A- 0 314 340**
**EP-A- 0 353 014**
**DE-A- 3 447 833**
**FR-A- 2 635 672**
**US-A- 2 980 941**

(73) Titulaire: **FINANCIERE ELYSEES BALZAC**
**2, Rue Balzac**
**F-75008 Paris (FR)**

(72) Inventeur: **Chalvin, Christophe**
**1, Rue Jacques de Guéhengnies**
**F-60000 Beauvais (FR)**
Inventeur: **Wertz, Jean-Luc**
**8, Rue Sainte-Angadrême**
**F-60000 Beauvais (FR)**
Inventeur: **Colin, Claudine**
**3, Rue Henri Barbusse**
**F-60290 Rantigny (FR)**

(74) Mandataire: **Le Roux, Martine et al**
**Cabinet Beau de Loménie**
**158, rue de l'Université**
**F-75340 Paris Cédex 07 (FR)**

## Description

La présente invention a pour objet un combiné, destiné notamment aux travaux de nettoyage et d'entretien ménager. Elle concerne également des procédés de fabrication dudit combiné.

Le combiné ou tampon de l'invention est composé d'un corps alvéolaire -en général, une éponge- et d'au moins un revêtement -en général, une nappe de fibres nontissée ou une peau- reliés par un joint de colle. Il contient, de façon caractéristique, des microcapsules susceptibles de libérer progressivement, au cours de son utilisation, le (ou les) principe(s) actif(s) qu'elles renferment.

On a, selon l'art antérieur, proposé différentes méthodes pour inclure des substances actives dans des combinés ou tampons, destinés à des travaux de nettoyage ou autres usages analogues.

On a notamment décrit :
- dans le brevet US-A-4,254,179, l'introduction de la matière active encapsulée dans la porosité d'un matériau en polyuréthanne ;
- dans le brevet US-A-3,334,374, l'introduction de microcapsules contenant une substance active liquide, entre deux couches de matériau. Ladite substance active est libérée par rupture de la membrane desdites microcapsules, sous l'action des pressions exercées par l'utilisateur ;
- dans la demande de brevet FR-A-2 358 864, l'introduction de la substance active dans une colle, déposée à la surface du matériau ; ladite colle assurant à la fois la fixation de la matière active et la libération contrôlée et retardée de celle-ci. L'adjonction de fines particules au mélange colle-matière active permet de modifier le profil de libération de ladite substance active.

Aucune de ces méthodes ne donne entière satisfaction. La mise en oeuvre des deux premières ne permet pas d'assurer un contrôle satisfaisant sur la libération de la matière active . La revendication principal part d'un état de l'art pas documenté.

La Demanderesse a présentement mis au point un combiné ou tampon, qui contient des substances actives, dont la libération peut être, à la fois, retardée et contrôlée.

Un tel combiné, comme indiqué ci-dessus, est composé d'un corps alvéolaire lié, sur au moins l'une de ses faces, à un revêtement par un joint de colle insoluble dans l'eau. Il se caractérise en ce qu'il contient au niveau dudit joint de colle mais, pas complètement noyées dans celui-ci, des microcapsules renfermant au moins un principe actif ; le matériau constituant les parois desdites microcapsules autorisant, uniquement en présence d'une quantité importante d'eau au sein du combiné, la libération dudit (ou desdits) principe(s) actif(s) par diffusion au travers desdites parois.

Le résultat escompté est donc obtenu, selon l'invention, grâce à l'intervention de microcapsules, qui renferment le (ou les) principe(s) actif(s) et qui ne le (les) libère(nt), par diffusion au travers de leur paroi, qu'en présence d'une quantité importante d'eau ; microcapsules qui, pour assurer ladite libération, ne doivent pas être complètement immergées dans le joint de colle.

La nature du matériau d'encapsulation et la localisation des microcapsules au sein du combiné constituent donc les deux caractéristiques essentielles des combinés de l'invention.

Au sein de ceux-ci, les microcapsules ne se trouvent donc immergées ni dans le corps alvéolaire, ni dans le revêtement, ni dans la colle. Elles se localisent au niveau du joint de colle, sans être totalement noyées dans ladite colle. On les trouve généralement aux interfaces joint de colle/corps alvéolaire et/ou joint de colle/revêtement. Lesdites microcapsules sont piégées mécaniquement, fixées par la colle mais cette dernière n'influe pas directement sur la cinétique de libération du (ou des) principe(s) actif(s) qu'elles renferment. Ladite cinétique est régie, par la diffusion, en présence d'eau, au travers des parois desdites microcapsules, en fait, au travers des portions de paroi, non noyées dans la colle.

Les caractéristiques essentielles de l'invention sont contenues dans la revendication 1. La revendication 9 décrit un procédé pour le production d'un combiné selon l'invention.

Avantageusement, le diamétre moyen des microcapsules est supérieur à l'épaisseur dudit joint de colle. On évite ainsi, aisément, lors de l'élaboration du combiné de l'invention, l'immersion totale desdites microcapsules dans ledit joint de colle.

La libération du (ou des) principe(s) actif(s) renfermé(s) dans les microcapsules se fait, au sein des combinés de l'invention par diffusion au travers des parois desdites microcapsules, uniquement en présence d'une quantité importante d'eau.

De nombreuses méthodes d'encapsulation de principe actif existent et sont parfaitement maîtrisées par l'homme du métier. La libération des principes actifs encapsulés peut se faire :
- par rupture de la membrane d'encapsulation ; rupture sous l'effet d'une pression, d'une tension, d'une force de contraction ou par abrasion ;
- par dégradation de ladite membrane ; dégradation par fusion, dissolution, décomposition thermique ou chimique ;
- par diffusion au travers de ladite membrane.

Les microcapsules intervenant dans les combinés de l'invention doivent libérer leur(s) principe(s) actif(s) selon la troisième de ces variantes ; compte tenu du mode d'utilisation desdits combinés et de la libération retardée et contrôlée, souhaitée pour le(s) principe(s) actif(s).

Par ailleurs, lesdites microcapsules doivent être en mesure de différer la diffusion jusqu'à la première utilisation du combiné dans lequel elles sont incluses.

Elles doivent retenir leur contenu au cours de la période s'étendant entre la fabrication du combiné et sa première utilisation, son premier contact avec une quantité importante d'eau. Il ne faut pas que ladite diffusion se fasse en cours de stockage du combiné. Or, au cours de ce stockage, le corps alvéolaire du combiné n'est généralement pas sec. Il est en effet habituel d'imprégner ledit corps alvéolaire, à la fin de son processus de fabrication, d'une solution plastifiante pour lui conférer une certaine souplesse. Une telle solution plastifiante consiste généralement en une solution aqueuse de chlorure de magnésium ou de dérivés du glycol.

Le choix du type de microcapsules -celui du matériau d'encapsulation et de la méthode d'encapsulation- intervenant dans les combinés de l'invention doit donc être guidé par les considérations ci-après :

- compatibilité du matériau d'encapsulation avec le (ou les) principe(s) actif(s) à encapsuler et avec les constituants des combinés (revêtement, corps alvéolaire, colle) ;
- libération du (ou des) principe(s) actif(s) par diffusion uniquement lors de la pénétration d'une quantité importante d'eau dans les combinés.

La localisation desdites microcapsules, au sein des combinés est par ailleurs, comme exposée ci-dessus, déterminante.

L'homme du métier est en mesure, au vu de ces considérations, de préparer les microcapsules adéquates.

Il mettra avantageusement en oeuvre des méthodes d'encapsulation basées sur la séparation de phases au sein d'une solution aqueuse de polymère(s) et notamment la méthode dite de coacervation, qui existe sous plusieurs variantes (coacervation simple, coacervation complexe, coacervation par effet de sel...).

Le procédé de base pour l'encapsulation par coacervation complexe est décrit dans le brevet US-A-2,800,457.

Ladite méthode comprend la dispersion du produit à encapsuler -hydrophobe- dans une solution aqueuse d'au moins un polymère hydrosoluble ; puis la séparation de ladite solution en deux phases, l'une riche en polymère(s) qui se dépose préférentiellement à la surface du produit à encapsuler, l'autre pauvre en polymère(s). Ladite séparation peut être provoquée de différentes manières: abaissement du pH, élévation de la température, interactions ioniques... Des post-traitements permettent éventuellement de solidifier la paroi des microcapsules obtenues.

De telles méthodes permettent notamment de préparer des microcapsules à partir de gélatine, agar-agar, pectine, méthyl cellulose, alcool polyvinylique ou fibrinogène... ; microcapsules utilisables dans les combinés de l'invention.

Les produits cités ci-dessus peuvent être utilisés en combinaison entre eux ou avec d'autres -notamment pour la mise en oeuvre d'une coacervation complexe- et générer des microcapsules dont les parois sont à base desdits produits mais peuvent également contenir lesdits autres produits.

La gélatine est un matériau d'encapsulation -utilisée seule ou majoritaire avec d'autres colloïdes polyanioniques tels que des polyphosphates, de la carboxyméthylcellulose, de l'alginate de sodium, des carraghénanes hydrolysés- qui convient particulièrement à la préparation de microcapsules, utilisables dans les combinés de l'invention. La préparation de telles microcapsules, à base de gélatine et de polyphosphates ou à base de gélatine et de polysaccharides anioniques, tel que par exemple des carraghénanes hydrolysés est notamment, respectivement, décrite dans le brevet US-A-3 697 437 et dans la demande EP-A-0 273 823.

La paroi de gélatine, après élaboration des microcapsules ne permet pas la diffusion de leur contenu. En revanche, au contact d'eau, ladite paroi gonfle et il est alors possible audit contenu de diffuser à travers celle-ci. Cette diffusion est ralentie, voire stoppée, lorsque le taux d'eau dans la paroi baisse en-dessous d'une certaine limite.

Ce phénomène s'est vérifié au sein des combinés de nettoyage de l'invention. Les microcapsules de gélatine, au cours de la phase de stockage qui peut s'étendre sur plusieurs mois, ne libèrent qu'une portion très faible de leur contenu, bien que le corps alvéolaire desdits combinés soit imprégné d'une solution plastifiante, alors que l'on constate une accélération du phénomène de libération si l'on plonge lesdites microcapsules dans un volume conséquent d'une solution identique à la solution plastifiante contenue dans ledit corps alvéolaire.

Avantageusement, le matériau constituant les parois des microcapsules que l'on trouve au niveau du joint de colle des combinés de l'invention est donc à base de gélatine.

Les inventeurs ont mis en évidence le fait que trois caractéristiques principales gouvernent la vitesse de libération par diffusion du contenu d'une microcapsule :

- le diamètre moyen de ladite microcapsule (D),
- le taux de matière active de celle-ci (T), défini par le rapport en pourcentage du poids de matière active sur le poids de capsule,

- l'épaisseur moyenne théorique de paroi (E), donnée par la formule :

$$E = \frac{D(1-T^{1/3})}{2}$$

Le diamètre moyen desdites microcapsules est fixé, en partie, comme indiqué ci-dessus, par l'épaisseur du joint de colle. Il est avantageusement compris entre 200 et 450 microns, de préférence voisin de 350 microns.

Il est évident par ailleurs que la paroi desdites microcapsules ne doit être ni trop fine, ni trop épaisse. Si elle est trop fine, elle présente une trop faible résistance mécanique et on observe alors un relargage immédiat du contenu des microcapsules. Si elle est trop épaisse, elle peut annihiler ledit relargage ou le ralentir dans une trop large mesure. En tout état de cause, la vitesse de libération de la matière active est d'autant plus lente que l'épaisseur moyenne théorique de paroi est plus grande.

Avantageusement, les combinés récurants de l'invention contiennent des microcapsules qui présentent une épaisseur moyenne théorique de paroi de l'ordre de 10 à 40 microns et plus préférentiellement comprise entre 14 et 35 microns, et un taux de matière active de 50 à 75 %.

Lesdites microcapsules renferment un (ou plusieurs) principe(s) actif(s). Le matériau encapsulé -liquide consistant en le(s) principe(s) actif(s) ou liquide contenant ledit (lesdits) principe(s) actif(s)- est avantageusement hydrophobe. Cette caractéristique lui permet d'être aisément encapsulé, notamment par les méthodes -de coacervation- évoquées ci-dessus.

Il peut s'agir d'une composition parfumante, d'un biocide, d'un solvant, d'un détachant... ou d'un mélange de ces produits. Cette liste n'est nullement exhaustive.

On notera qu'un combiné de l'invention peut contenir des microcapsules renfermant un unique principe actif, des microcapsules renfermant un mélange de principes actifs et/ou un mélange de microcapsules renfermant des principes actifs différents. On veillera avantageusement, en tout état de cause, à ce que le matériau encapsulé -appelé à être libéré- soit compatible avec la colle, le corps alvéolaire et son revêtement.

Ainsi, selon une variante préférée, le combiné de l'invention contient des microcapsules qui renferment une composition parfumante. On évitera d'utiliser des compositions parfumantes, susceptibles de colorer tout ou partie du combiné, susceptibles de virer par réaction chimique avec la solution plastifiante contenue dans le corps alvéolaire...

Ledit corps alvéolaire consiste généralement en une mousse polyuréthanne ou une éponge végétale à base de cellulose régénérée. Il peut consister en une éponge naturelle mais on sait que de telles éponges sont à priori réservées à d'autres applications...

Les propriétés de la cellulose régénérée rendent particulièrement intéressant l'emploi d'éponges végétales pour la fabrication de combinés selon l'invention.

Comme indiqué ci-dessus, ledit corps alvéolaire est généralement à la fin de son processus de fabrication, imprégné d'une solution plastifiante qui lui confère une certaine souplesse.

Ledit corps alvéolaire est recouvert, sur au moins l'une de ses faces, d'un revêtement. Ledit revêtement peut notamment consister en une nappe de fibres nontissée ou en une peau, synthétique ou de chamois.

La nappe de fibres nontissée se présente généralement sous la forme d'une nappe de moins de 1 cm d'épaisseur, constituée de fibres liées entre elles par une résine. Eventuellement, cette nappe contient des grains d'une substance abrasive. Des procédés de fabrication de telles nappes sont décrits notamment dans les brevets FR 1 239 913, 1 256 972, 1 284 723 et 2 253 488.

L'association selon l'invention du corps alvéolaire et d'une telle nappe génère des combinés récurants, renfermant des microcapsules.

Ledit corps alvéolaire peut aussi être recouvert, sur au moins l'une de ses faces, d'une peau. Il peut s'agir d'une peau naturelle (peau de chamois) ou synthétique (peau monoface ou biface). De telles peaux ainsi que leur association à des éponges cellulosiques ou mousses polyuréthanne ont été décrites dans l'art antérieur, notamment dans les brevets GB 1 092 902, BE 525 759 et US 3,083,392. Une peau monoface résulte généralement de la superposition d'un matériau rétenteur d'eau (par exemple un nontissé) et d'un matériau poreux (mousse de latex, mousse polyuréthanne ) ; une peau biface de la prise en sandwich d'un tel matériau rétenteur d'eau par un tel matériau poreux.

L'association selon l'invention du corps alvéolaire et d'une telle peau génère des combinés essuyants, renfermant des microcapsules.

L'invention a plus particulièrement pour objet :
- des combinés récurants composés d'un corps alvéolaire et d'une nappe de fibres nontissée, liés par un joint de colle contenant les microcapsules adéquates ; combinés : corps alvéolaire/ colle/nappe de fibres nontissée ; et
- des combinés essuyants composés d'un corps alvéolaire et d'une peau, liés par un joint de colle contenant les microcapsules adéquates ; combiné : corps alvéolaire/colle/peau.

Elle englobe toutefois d'autres variantes, et notamment des combinés symétriques :

- nappe de fibres nontissée/colle/corps alvéolaire/colle/nappe de fibres nontissée
- peau/colle/corps alvéolaire/colle/peau et des combinés dissymétriques :
- nappe de fibres nontissée/colle/corps alvéolaire/colle/peau.

De tels combinés selon l'invention contiennent des microcapsules telles que définies ci-dessus au niveau d'au moins un de leurs joints de colle.

La colle assure la cohésion des combinés de l'invention. Divers types de colle peuvent être employés pour assembler le corps alvéolaire et son revêtement, notamment :

- des colles en milieu solvant (par exemple : copolymère butadiène styrène acrylonitrile carboxylé dissout dans la méthyléthylcétone) ;
- des colles sans solvant (par exemple : des polyuréthannes mono- ou bi-composants).

On veille évidemment à respecter les diverses compatibilités : colle/matériau d'encaosulation, colle/principe actif encapsulé. On utilise la quantité de colle adéquate pour assurer une bonne cohérence des combinés de l'invention.

La quantité de colle à utiliser pour obtenir une bonne cohérence d'un combiné récurant (corps alvéolaire/colle/nappe de fibres nontissée) est généralement de 200 grammes par m² de nappe de nontissé. L'épaisseur moyenne du film de colle est voisine de 200 microns.

Selon une variante préférée, le combiné de l'invention est un combiné récurant, composé d'un matériau alvéolaire cellulosique -éponge, obtenue par le procédé viscose- imprégné d'une solution plastifiante et d'une nappe de fibres nontissée récurante, liés par un joint de colle. La solution plastifiante intervenant est avantageusement une solution aqueuse de chlorure de magnésium ; la colle utilisée, une colle de type polyuréthanne. Ledit combiné contient, au niveau de la face de contact de son joint de colle et de ladite nappe de fibres nontissée récurante des microcapsules de gélatine, renfermant une composition parfumante. On trouve généralement environ 45 grammes desdites microcapsules par m² de nontissé.

Un tel combiné récurant parfumé ne libère son parfum progressivement qu'à l'usage.

Différents procédés peuvent être mis en oeuvre pour la fabrication des combinés de l'invention.

Nous nous proposons d'en développer ci-après quelques uns ; leurs mises en oeuvre ne soulèvent aucun problème particulier.

Le procédé de fabrication d'un combiné type corps alvéolaire/colle/revêtement peut se dérouler en trois étapes :

- enduction de la colle,
- dépose des microcapsules à la surface de la colle,
- réalisation du complexe corps alvéolaire/revêtement.

Un tel procédé englobe deux variantes.

Selon la première, on enduit de colle une face du corps alvéolaire ; on dépose les microcapsules sur cette face encollée. On met au contact de cette face encollée du corps alvéolaire le revêtement et l'ensemble est maintenu sous pression durant la durée nécessaire à la prise de la colle. Le corps alvéolaire se présente généralement à l'enducteur de colle sous forme de plaques, sur un tapis roulant ; le dispositif utilisé pour le poudrage des microcapsules doit être en mesure de déposer celles-ci de façon homogène. De tels dispositifs existent et sont connus de l'homme du métier.

Selon la seconde, on enduit de colle une face du revêtement ; on dépose les microcapsules sur cette face encollée ; on met au contact de cette face encollée du revêtement, le corps alvéolaire et l'ensemble est maintenu sous pression durant la durée nécessaire à la prise de la colle. Le revêtement est dirigé généralement vers l'enducteur de colle sur un tapis roulant ; la dépose des microcapsules se fait comme indiquée ci-dessus par un dispositif ad-hoc.

Le complexe formé est généralement, selon ces deux variantes, enroulé sous une tension déterminée, afin de permettre une bonne adhésion du revêtement à la surface du corps alvéolaire.

D'autres procédés de fabrication peuvent être mis en oeuvre pour l'élaboration de ce même type de combinés de l'invention, notamment un procédé englobant les étapes ci-après :

- éventuelle dépose de microcapsules sur une face du corps alvéolaire ou du revêtement ;
- pulvérisation de colle sur ladite face ou sur une face vierge du corps alvéolaire ou du revêtement ;
- dépose de microcapsules sur la couche de colle ;
- réalisation du complexe corps alvéolaire/revêtement.

Ce procédé englobe, de la même façon, deux variantes principales, selon que l'on choisit d'effectuer la pulvérisation de la colle sur une face du corps alvéolaire ou sur une face du revêtement. Il englobe évidemment d'autres variantes selon que l'on opte pour une ou deux déposes de microcapsules. La pulvérisation de colle se fait de manière connue, au pistolet par exemple.

D'autres procédés sont également envisageables, avec enduction ou pulvérisation de colle sur une face de chacun des constituants de base des combinés : corps alvéolaire/revêtement.

Les procédés ci-dessus et leurs variantes font partie intégrante de la présente invention.

Après contre-collage et prise de la colle, les combinés obtenus peuvent être découpés à la scie ou par emporte-pièces, en fonction de la complexité des formes à réaliser.

La préparation de combinés symétriques ou dissymétriques, présentant deux joints de colle, se déduit à l'évidence des procédés connus de contre-collage et des modes d'incorporation des microcapsules, décrits ci-dessus.

Avantageusement, on prépare de tels combinés, en deux étapes successives :
- élaboration d'un premier combiné : corps alvéolaire/ revêtement ;
- reprise de celui-ci, avant découpe, pour traitement de l'autre face dudit corps alvéolaire et obtention d'un combiné : revêtement / corps alvéolaire / revêtement ou revêtement / corps alvéolaire / revêtement ;
l'incorporation de microcapsules intervenant au cours d'au moins l'une de ses étapes.

L'invention a été mise au point et testée, notamment avec des microcapsules contenant des compositions parfumantes.

Deux tests ont été développés afin de contrôler l'efficacité du parfumage.

Le premier d'entre eux nommé "test vaisselle" consiste à faire subir au combiné parfumé cinq séjours d'une heure dans de l'eau tiède additionnée de tensio-actif à raison d'un séjour par jour. Un panel interne détermine alors si le parfum peut encore être perçu. Les microcapsules sont évaluées en fonction du nombre de cycles donnant un résultat positif que peuvent subir les combinés.

Le second dit "test machine à laver" consiste à faire subir au combiné parfumé un cycle machine à laver dans des conditions définies (60 °C, présence ou absence de détergent, essorage 500 tours/minute). De même un panel interne détermine alors si le parfum peut encore être perçu.

Par ailleurs, afin de déterminer si les caractéristiques des microcapsules n'étaient pas affectées par un temps de stockage important en présence de chlorure de magnésium dans l'emballage non étanche utilisé par la Demanderesse, un test de vieillissement accéléré a été mis au point. Il consiste à introduire durant 120 heures les produits dans une étuve à 40 °C, saturée en humidité, puis à les soumettre à un rayonnement U.V. durant 48 heures. On vérifie après 4 à 8 cycles que les propriétés du produit n'ont pas été affectées.

L'évaluation des combinés de l'invention a montré que, suivant les compositions parfumantes retenues, le parfum était encore perceptible après 2 à 4 tests vaisselle, après 1 cycle machine à laver, et qu'après 4 cycles de vieillissement, on n'observait pas de modification notable desdits produits.

D'autre part, il a été constaté que l'utilisation selon l'invention de microcapsules de compositions parfumantes permettait d'augmenter de façon sensible la durée pendant laquelle le parfum est perceptible ; ceci en comparant des combinés contenant la même quantité de composition parfumante, les uns sous forme microencapsulée, les autres sous forme liquide (le parfum sous forme liquide est injecté, au moyen d'une seringue, au niveau du joint de colle de combinés ne contenant pas de microcapsules).

L'invention est illustrée par les exemples ci-après.

EXEMPLE 1

Un tampon récurant parfumé est réalisé à partir des matières premières suivantes :
- une éponge cellulosique, obtenue par le procédé viscose et présentant les caractéristiques suivantes :
  . épaisseur : 23 mm
  . densité (rapport du poids de l'éponge séchée au volume de l'éponge humide après centrifugation à 3 000 tours/mn durant 2 mn) : 0,03
  . imprégnée, à la fin de son cycle de fabrication, de 260 grammes d'une solution plastifiante contenant 14,4 % (en poids) de chlorure de magnésium anhydre pour 100 g de cellulose sèche ;
- une nappe de nontissé récurante présentant les caractéristiques suivantes :
  . épaisseur : environ 5 mm
  . grammage : 350 grammes par m$^2$ environ ;
- de la colle polyuréthanne monocomposant (qui réticule sous la seule action de l'eau) ;
- des microcapsules qui présentent les caractéristiques suivantes :
  . granulométrie moyenne : 305 microns
  . taux de matière active : 51 %
  . épaisseur théorique moyenne de paroi : 31 microns
  . matière active : composition parfumante (liquide mobile jaune pâle, accord hespéridé, herbal, fleuri et boisé) et qui sont obtenues par coacervation complexe de gélatine et de polyphosphates.

La gélatine utilisée présente une force en gelée de 225 ± 10 blooms (force en gelée, mesurée par la méthode décrite dans le British Standard).

Les polyphosphates utilisés sont ceux commercialisés sous la marque Hexatren® par la société GIULINI CHEMIE GmbH.

Ledit tampon est réalisé à partir desdites matières premières comme indiqué ci-après.

Le rouleau de nontissé récurant est déroulé sur un tapis roulant. La nappe de nontissé est dirigée vers l'enducteur qui dépose 200 grammes de colle par m² dudit nontissé récurant.

Les microcapsules sont alors déposées à raison de 45 grammes par m² de nontissé récurant, sur la couche de colle (dont l'épaisseur est voisine de 200 microns).

Les plaques d'éponges sont posées à la surface du nontissé encollé et l'ensemble est maintenu sous pression, par enroulement sous une tension déterminée.

Les combinés sont ensuite découpés à l'emporte-pièces aux dimensions souhaitées ; par exemple : 71 x 111 mm.

De tels combinés contiennent environ 350 mg de microcapsules en leur sein, soit 180 mg de composition parfumante.

On a par ailleurs réalisé, à titre comparatif, des tampons quasi-identiques (même corps alvéolaire, même nappe de nontissé assemblés, comme indiqué ci-dessus avec la même colle) sans microcapsules. On a introduit dans ceux-ci sous forme liquide (par injection au niveau du joint de colle) 180 mg de la même composition parfumante.

La rémanence du parfum est, après réalisation des combinés, beaucoup plus importante dans les combinés contenant les microcapsules.

Le parfum est encore perceptible au sein de ceux-ci, après 3 cycles vaisselle.

En revanche, il n'est plus du tout perceptible après un cycle vaisselle pour les combinés au sein desquels il avait été introduit sous forme liquide.

EXEMPLE 2

On réalise un tampon récurant parfumé, comme à l'exemple 1, à partir des mêmes matières premières -éponge cellulosique, nappe de nontissé récurante, colle polyuréthanne-. On utilise toutefois des microcapsules qui présentent les caractéristiques suivantes :
- granulométrie moyenne : 419 microns
- taux de matière active : 73,7 %
- épaisseur théorique moyenne de paroi : 20 microns
- matière active : composition parfumante (liquide mobile jaune pâle, accord hespéridé, herbal, fleuri et boisé)
  et qui sont obtenues par coacervation complexe de gélatine et de carraghénane hydrolysé.

On utilise, comme à l'exemple 1, une gélatine présentant une force en gelée de 225 ± 10 blooms.

Le carraghénane utilisé est commercialisé sous la marque Stabilgum ® par la société SANOFI BIO INDUSTRIES. Il présente une viscosité de 10 à 20 mP.s (viscosité d'une solution aqueuse à 15 g/l, mesurée à 75°C avec un viscosimétre rotatif Brookfield de type LVT équipé d'un mobile UL à vitesse de rotation de 6 tours/minute).

Les combinés obttenus contiennent environ 350 mg de microcapsules en leur sein, soit 225 mg de composition parfumante.

EXEMPLE 3

On réalise un tampon récurant parfumé, comme à l'exemple 1, à partir des mêmes matières premières -éponge cellulosique, nappe de nontissé récurante, colle polyuréthanne-. On utilise toutefois des microcapsules qui présentent les caractéristiques suivantes :
- granulométrie moyenne : 284 microns
- taux de matière active : 72 %
- épaisseur théorique moyenne de paroi : 15 microns
- matière active : composition parfumante (liquide mobile jaune, accord hespéridé)
  et qui sont obtenues par coacervation complexe de gélatine et de carraghénane hydrolysé (la gélatine et le carraghénane utilisé sont les mêmes que ceux de l'exemple 2).

Les combinés obtenus contiennent environ 350 mg de microcapsules en leur sein, soit 250 mg de composition parfumante.

Au sein desdits combinés, le parfum est encore perceptible, après 4 cycles vaisselle.

**Revendications**

1. Combiné de nettoyage, composé d'un corps alvéolaire du type mousse polyuréthanne ou éponge lié, sur au moins l'une de ses faces, à un revêtement du type nappe de fibres nontissée ou peau synthétique ou de chamois, par un joint de colle insoluble dans l'eau, caractérisé en ce qu'il contient au niveau dudit joint de colle, mais pas complètement noyées dans celui-ci, des microcapsules renfermant au moins un principe actif; le matériau constituant les parois desdites microcapsules autorisant la libération dudit (ou desdits) principe(s) actif(s) par diffusion au travers desdites parois, uniquement en présence d'une quantité importante d'eau au sein dudit combiné.

2. Combiné selon la revendication 1, caractérisé en ce que le diamètre moyen desdites microcapsules est supérieur à l'épaisseur dudit joint de colle.

3. Combiné selon l'une des revendications 1 ou 2, caractérisé en ce que le matériau constituant les parois desdites microcapsules est à base de gélatine.

**4.** Combiné selon l'une quelconque des revendications 1 à 3, caractérisé en ce que lesdites microcapsules ont une granulométrie moyenne comprise entre 200 et 450 microns.

**5.** Combiné selon l'une quelconque des revendications 1 à 4, caractérisé en ce que lesdites microcapsules renferment un taux de principe actif compris entre 50 et 75%.

**6.** Combiné selon l'une quelconque des revendications 1 à 5, caractérisé en ce que lesdites microcapsules renferment une composition parfumante, un biocide, un solvant, un détachant ou un mélange desdits principes actifs.

**7.** Combiné de nettoyage selon l'une quelconque des revendications précédentes caractérisé en ce que son corps alvéolaire consiste en une éponge végétale à base de cellulose régénérée, avantageusement imprégnée d'une solution plastifiante.

**8.** Combiné selon la revendication 1, composé d'un matériau alvéolaire cellulosique imprégné d'une solution plastifiante et d'une nappe de fibres nontissée récurante, liés par un joint de colle, caractérisé en ce qu'il contient au niveau de la face de contact dudit joint de colle et de ladite nappe de fibres nontissée récurante des microcapsules de gélatine, renfermant une composition parfumante ; à raison d'environ 45 grammes desdites microcapsules par $m^2$ de nontissé.

**9.** Procédé de fabrication d'un combiné selon l'une quelconque des revendications 1 à 8, caractérisé en ce qu'il inclut les étapes :
   - d'enduction de colle d'une face dudit corps alvéolaire ou d'une face dudit revêtement ;
   - de dépose à la surface de la couche de colle des micro-capsules adéquates ;
   - et de mise en contact de ladite face encollée dudit corps alvéolaire ou dudit revêtement sur laquelle ont été déposées lesdites microcapsules avec la face correspondante -vierge- de l'autre composant du combiné, à savoir respectivement la face dudit revêtement ou dudit corps alvéolaire.

**10.** Procédé de fabrication d'un combiné selon l'une quelconque des revendications 1 à 8, caractérisé en ce qu'il inclut les étapes de :
   - pulvérisation de colle sur une face dudit corps alvéolaire ou dudit revêtement ; des microcapsules adéquates ayant été éventuellement préalablement déposées sur ladite face ;
   - de dépose de microcapsules adéquates à la surface de la couche de colle ;
   - et de mise en contact de ladite face portant les microcapsules et la colle avec la face correspondante -vierge- de l'autre composant du combiné, à savoir respectivement une face dudit revêtement ou dudit corps alvéolaire.

**Claims**

**1.** Cleaning combination, composed of a cellular body of the polyurethane foam or sponge type bonded, on at least one of its faces, to a covering of the non-woven sheet of fibres or synthetic skin or chamois type by a water-insoluble adhesive seal, characterized in that it contains at the level of the said adhesive seal, but not completely embedded in the latter, microcapsules containing at least one active principle, the material constituting the walls of the said microcapsules allowing the release of the said active principle(s) by diffusion through the said walls, solely in the presence of a significant amount of water in the said combination.

**2.** Combination according to Claim 1, characterized in that the mean diameter of the said microcapsules is greater than the thickness of the said adhesive seal.

**3.** Combination according to either of Claims 1 and 2, characterized in that the material constituting the walls of the said microcapsules is based on gelatin.

**4.** Combination according to any one of Claims 1 to 3, characterized in that the said microcapsules have a mean particle size of between 200 and 450 microns.

**5.** Combination according to any one of Claims 1 to 4, characterized in that the said microcapsules contain an active principle level of between 50 and 75%.

**6.** Combination according to any one of Claims 1 to 5, characterized in that the said microcapsules contain a perfuming composition, a biocide, a solvent, a stain remover or a mixture of the said active principles.

**7.** Cleaning combination according to any one of the preceding claims, characterized in that its cellular body comprises a vegetable sponge based on regenerated cellulose, advantageously impregnated with a plasticizing solution.

8. Combination according to Claim 1, composed of a cellulose cellular material impregnated with a plasticizing solution and of a scouring non-woven sheet of fibres bonded by an adhesive seal, characterized in that it contains, at the contact face of the said adhesive seal and the said scouring non-woven sheet of fibres, gelatin microcapsules containing a perfuming composition, in a proportion of approximately 45 grams of the said microcapsules per m$^2$ of non-woven material.

9. Process for the manufacture of a combination according to any one of Claims 1 to 8, characterized in that it includes the stages:
   - of covering a face of the said cellular body or a face of the said covering with adhesive;
   - of depositing appropriate microcapsules at the surface of the layer of adhesive;
   - and of bringing the said adhesive-covered face of the said cellular body or of the said covering on which the said microcapsules have been deposited into contact with the corresponding "virgin" face of the other component of the combination, namely respectively the face of the said covering or of the said cellular body.

10. Process for the manufacture of a combination according to any one of Claims 1 to 8, characterized in that it includes the stages:
    - of spraying adhesive onto a face of the said cellular body or of the said covering, appropriate microcapsules having optionally been deposited beforehand on the said face;
    - of depositing appropriate microcapsules at the surface of the layer of adhesive;
    - and of bringing the said face carrying the microcapsules and the adhesive into contact with the corresponding "virgin" face of the other component of the combination, namely respectively a face of the said covering or of the said cellular body.

**Patentansprüche**

1. Reinigungsvorrichtung, bestehend aus einem zellenförmigen Körper aus PUR-Elastomerschaum oder Schwamm, der an mindestens einer seiner Seiten durch eine wasserunlösliche Klebverbindung mit einer Vliesfaserpelz- oder Kunstleder- oder Waschlederbeschichtung verbunden ist,
dadurch gekennzeichnet, daß sie im Bereich der genannten Klebverbindung, aber nicht gänzlich in dieser versenkt, Mikrokapseln aufweist, die mindestens einen Wirkstoff beinhalten, wobei das Material, aus dem die Wände der genannten Mikrokapseln bestehen, das Freisetzen des (oder der) genannten Wirkstoffs (oder Wirkstoffe) durch Ausbreitung durch die genannten Wände hindurch nur dann zuläßt, wenn sich eine bedeutende Menge Wasser an der genannten Reinigungsvorrichtung befindet.

2. Reinigungsvorrichtung nach Anspruch 1, dadurch gekennzeichnet, daß der durchschnittliche Durchmesser der genannten Mikrokapseln größer ist als die Dicke der genannten Klebverbindung.

3. Reinigungsvorrichtung nach einem der Ansprüche 1 oder 2, dadurch gekennzeichnet, daß das Material, aus dem die Wände der genannten Mikrokapseln bestehen, auf Gelatinebasis hergestellt ist.

4. Reinigungsvorrichtung nach einem der Ansprüche 1 bis 3, dadurch gekennzeichnet, daß die genannten Mikrokapseln eine durchschnittliche Korngroße zwischen 200 und 450 Mikrometern besitzen.

5. Reinigungsvorrichtung nach einem der Ansprüche 1 bis 4, dadurch gekennzeichnet, daß die genannten Mikrokapseln einen Wirkstoffanteil von 50 bis 75 % beinhalten.

6. Reinigungsvorrichtung nach einem der Ansprüche 1 bis 5, dadurch gekennzeichnet, daß die genannten Mikrokapseln eine parfümierende Zusammensetzung, ein Biozid, ein Losungsmittel, ein Detachiermittel oder ein Gemisch der genannten Wirkstoffe beinhalten.

7. Reinigungsvorrichtung nach einem der vorherigen Ansprüche, dadurch gekennzeichnet, daß ihr zellenförmiger Körper aus einem pflanzlichen Schwamm auf Basis von Regeneratzellulose besteht, der vorteilhafterweise mit einer Plastizierlösung getränkt ist.

8. Reinigungsvorrichtung nach Anspruch 1, bestehend aus einem zellenförmigen Zellulosematerial, das mit einer Plastizierlösung getränkt ist, und aus einem Vliesfaserpelz zum Scheuern, die durch eine Klebverbindung miteinander verbunden sind,
dadurch gekennzeichnet, daß sie im Bereich der Berührungsseite der genannten Klebverbindung und des genannten Vliesfaserpelzes zum Scheuern Gelatinemikrokapseln aufweist, die eine parfümierende Zusammensetzung beinhalten, und zwar im Verhältnis von etwa 45 g der genannten Mikrokapseln pro m$^2$ Vliesfaserpelz.

9. Herstellungsverfahren für eine Reinigungsvorrichtung nach einem der Ansprüche 1 bis 8, dadurch gekennzeichnet, daß es die folgenden Schritte umfaßt:
   - Bestreichen einer Seite des genannten zellenförmigen Körpers oder einer Seite der genannten Beschichtung mit Klebstoff,
   - Ablegen von geeigneten Mikrokapseln auf der Seite mit der Klebschicht,
   - und Zusammenfügen der genannten mit Klebstoff versehenen Seite des genannten zellenförmigen Körpers oder der genannten Beschichtung, auf der die genannten Mikrokapseln abgelegt wurden, mit der entsprechenden - unberührten - Seite des jeweils anderen Bestandteils der Kombination, d. h. der Seite der genannten Beschichtung oder des genannten zellenförmigen Körpers.

10. Herstellungsverfahren für eine Reinigungsvorrichtung nach einem der Ansprüche 1 bis 8, dadurch gekennzeichnet, daß es die folgenden Schritte umfaßt.
   - Zerstäuben von Klebstoff auf einer Seite des genannten zellenförmigen Körpers oder der genannten Beschichtung, wobei eventuell zuvor geeignete Mikrokapseln auf der genannten Seite abgelegt wurden.
   - Ablegen von geeigneten Mikrokapseln auf der Seite mit der Klebschicht,
   - und Zusammenfügen der genannten Seite, auf der sich die Mikrokapseln und der Klebstoff befinden, mit der entsprechenden - unberührten - Seite des jeweils anderen Bestandteils der Kombination, d. h. einer Seite der genannten Beschichtung oder des genannten zellenförmigen Körpers.